# EUROPÄISCHE PATENTSCHRIFT

(11) **EP 1 241 478 B1**
(45) Veröffentlichungstag und Bekanntmachung des Hinweises auf die Patenterteilung: **20.12.2006**
(21) Anmeldenummer: 02005564.6
(22) Anmeldetag: 12.03.2002
(51) Int. Cl.: G01N 33/68, G01N 33/569, G01N 33/84, C12Q 1/37, C12Q 1/527, C12Q 1/32, C12Q 1/48, C12Q 1/42, C12Q 1/68

(54) **Verfahren zur Erfassung der Pathogenese und/oder zur Diagnose von 'transmissiblen spongiformen Enzephalopathien'**
Method for detecting the pathogenesis and/or diagnosing transmissible spongiform encephalopathies
Méthode pour la détection de la pathogenèse et/ou le diagnostic des encéphalopathies spongiformes transmissibles

(30) Priorität: 12.03.2001 DE 10112097
(43) Veröffentlichungstag der Anmeldung: 18.09.2002
(73) Patentinhaber: Menzel, Ingrid, Dr., 61348 Bad Homburg v.d. H. (DE)
(72) Erfinder: Menzel, Ingrid, Dr., 61348 Bad Homburg v.d. H. (DE)
(74) Vertreter: Manitz, Finsterwald & Partner GbR

(56) Entgegenhaltungen:
- WO-A-00/29850
- WO-A-00/31545
- WO-A-98/13694
- WO-A-99/47932
- US-A- 4 678 748
- US-A- 4 772 551
- US-A- 5 409 814
- PRATT D E ET AL: "FAST ATOM BOMBARDMENT MASS SPECTROMETRY METHOD FOR MONITORING ZINC AND CALCIUM STABLE ISOTOPES IN BIOAVAILABILITY STUDIES" JOURNAL OF MICRONUTRIENT ANALYSIS, Bd. 3, Nr. 2, 1987, Seiten 107-118, XP008030628 ISSN: 0266-349X
- DANKS D M: "DIAGNOSIS OF TRACE METAL DEFICIENCY WITH EMPHASIS ON COPPER AND ZINC" AMERICAN JOURNAL OF CLINICAL NUTRITION, Bd. 34, Nr. 2, 1981, Seiten 278-280, XP008030549 ISSN: 0002-9165
- LIU REN-MIN ET AL: "Simultaneous determination of multicomponents by flow injection analysis" TALANTA, Bd. 40, Nr. 4, 1993, Seiten 511-514, XP008030621 ISSN: 0039-9140
- AHLSKOG J E ET AL: "Guamanian neurodegenerative disease: Investigation of the calcium metabolism/heavy metal hypothesis" NEUROLOGY, Bd. 45, Nr. 7, 1995, Seiten 1340-1344, XP008030551 ISSN: 0028-3878
- BELLISOLA G ET AL: "Use of total-reflection X-ray fluorescence to track the metabolism and excretion of selenium in humans" PROCEEDINGS OF THE 1998 7TH CONFERENCE ON TOTAL REFLECTION X-RAY FLUORESCENCE ANALYSIS AND RELATED METHODS (TXRF'98);AUSTIN, TX, USA SEP 27-OCT 3 1998, Bd. 54, Nr. 10, 27. September 1998 (1998-09-27), Seiten 1481-1485, XP002280018 Spectrochim. Acta Part B At. Spectrosc.;Spectrochimica Acta - Part B Atomic Spectroscopy 1999 Elsevier Science B.V., Amsterdam, Netherlands
- WARRELL R P JR ET AL: "PHASE I EVALUATION OF SUCCINYLATED ACINETOBACTER-GLUTAMINASIFICANS GLUTAMINASE ASPARAGINASE IN ADULTS" CANCER RESEARCH, Bd. 40, Nr. 12, 1980, Seiten 4546-4551, XP008030123 ISSN: 0008-5472

## Beschreibung

Die vorliegende Erfindung betrifft ein Verfahren zur Erfassung der Pathogenese und zur Diagnose von 'transmissiblen spongiformen Enzephalopathien' bei Menschen und Vertebraten, insbesondere zur Diagnose der 'Bovinen Spongiformen Enzephalopathie' bei Rindern und zur Diagnose der 'Creutzfeldt-Jakob-Krankheit' bei Menschen, bei welchem dem zu untersuchenden Individuum eine biologische Probe entnommen und deren Gehalt an Neurotoxinen, insbesondere an neurotoxischen Mykotoxinen, und/oder an neurotoxinbildenden Mikroorganismen, beispielsweise an Hefe oder an Schimmelpilzen, und/oder an Zink bestimmt wird.

Bei den 'transmissiblen spongiformen Enzephalopathien' (TSEs) handelt es sich um übertragbare Erkrankungen des Zentralnervensystems, die durch Degeneration von Nervenzellen nach kurzer Zeit zum Tod führen. Von der Degeneration besonders betroffen sind bestimmte Bereiche der grauen Gehirnsubstanz, die mikroskopisch schwammartige Veränderungen aufweisen. Ein weiteres Kennzeichen für die TSEs sind Ablagerungen unlöslicher Proteinen im Gehirn, sogenannten "amyloide Plaques".

Einige der zu dieser Gruppe zählenden Krankheiten sind bereits seit Jahrhunderten bekannt, wie beispielsweise die erstmals im 18. Jahrhundert bei Schafen beschriebene und inzwischen diagnostizierte Krankheit 'Scrapie'. Die von dieser Krankheit befallenen Schafe zeigen auffällige Verhaltensstörungen und leiden an so starkem Juckreiz, dass sie sich das Fell abkratzen. Zu der Gruppe der TSEs zählt auch die seit 1985 bekannte Rinderkrankheit 'Bovine Spongiform Encephalopathy' (BSE), die auffallend ängstliche und aggressive Kühe hervorbringt, welche im späteren Verlauf der Krankheit Bewegungsstörungen zeigen und schließlich zusammenbrechen. Inzwischen ist diese Krankheit bei mehr als 200.000 Rindern, überwiegend in Westeuropa, diagnostiziert worden.

Beim Menschen werden zu der Gruppe der TSEs die seit der Jahrhundertwende bekannte und nach fortschreitendem geistigen Verfall und massiven Bewegungsstörungen innerhalb von wenigen Monaten zum Tod führende Creutzfeldt-Jakob-Krankheit (CJK), das Gerstmann-Sträussler-Scheinker-Syndrom (GSS) und die vermutlich durch das rituelle Verspeisen von Hirn der Verstorbenen bei einem Eingeborenen-Stamm in Papua-Neuguinea verbreitete Krankheit Kuru gezählt.

Abgesehen von den prinzipiell gleichen Symptomen und dem immer tödlichen Verlauf der Krankheiten, sind alle TSEs durch die lange, in der Regel mehrjährige symptomfreie Inkubationszeit zwischen Infektion und Ausbruch der Krankheit gekennzeichnet, was auf einen prinzipiell gleichartigen Pathomechanismus aller Krankheiten schließen lässt. Ein weiteres Indiz für einen gleichartigen Erreger ist die experimentell belegte Übertragbarkeit von einigen TSEs über die Artenbarriere hinweg. So erfolgte z. B. durch Verfütterung von pathologischer Gehirnsubstanz eines BSE-kranken Rindes die experimentelle Übertragung von BSE auf Schafe.

Nicht zuletzt aufgrund der Ausbreitung von BSE und dem vermuteten Zusammenhang von BSE- und CJK-Fällen bei Menschen ist ein schnell und zuverlässig durchzuführendes Verfahren zur eindeutigen Diagnose von TSEs dringend erforderlich. Bisher beruht die klinische Diagnose von BSE und CJK weitgehend auf neurologischen Beobachtungen, so dass eine definitive Diagnose erst nach dem Tod am Gehirn möglich ist. Um die Rinderseuche BSE einzudämmen und CJK bei Menschen frühzeitig zu erkennen, ist jedoch ein bei lebenden Individuen anwendbares, möglichst zuverlässig durchführbares Diagnoseverfahren notwendig. Ein solches Verfahren setzt genaue Kenntnisse über die Ursachen bzw. den Erreger der TSEs voraus.

Trotz intensiver Forschung ist die Natur der TSE-Erreger nicht geklärt, es liegt jedoch die gesicherte Erkenntnis vor, dass es sich bei diesem um einen sehr stabilen Erreger handelt. Die Stabilität des Erregers manifestiert sich insbesondere in seiner Resistenz gegenüber formaldehyd- und alkoholhaltigen Desinfektionsmitteln, gegenüber ultravioletter und ionisierter Strahlung sowie gegenüber Proteasen. Aufgrund dieser großen Stabilität wurde bereits in den 70er Jahren vermutet, dass es sich bei den Erregern nicht um bekannte nukleinsäurehaltige Erreger wie Bakterien oder Viren handelt, sondern um reine Proteine. Diese These wird vor allem darauf gestützt, dass in den Gehirnen erkrankter Menschen und Tiere eine gegenüber dem nativen, in den Hirnzellen von Menschen und Vertebraten vorkommenden Prion-Protein (PrP^{c}) modifizierte Form dieses Proteins (PrP^{Sc}) gefunden wird. Das modifizierte Prion-Protein ist dem nativen Prion-Protein hinsichtlich der Aminosäuresequenz und dem Glykosylierungsmuster identisch und unterscheidet sich von diesem vornehmlich durch seine Resistenz gegenüber Proteasen, seine thermische Resistenz, seine Stabilität gegenüber chemischen Agenzien sowie in der Agglomerisierungstendenz und Unlöslichkeit in nicht denaturierenden Detergentien. Diese unterschiedlichen Stabilitäten beider Proteine ist auf die durch physikalische Untersuchungen belegte unterschiedliche Sekundär- und Tertiärstruktur beider Proteine zurückzuführen; so besteht PrP^{Sc} hauptsächlich aus β-Faltblattstrukturen, während PrP^{c} vornehmlich α-helikale Strukturen aufweist. Trotz der eine enge Assoziation zwischen dem Prion-Protein und den TSEs-Erkrankungen nahe legenden Erkenntnisse, ist nicht bewiesen, dass PrP^{Sc} der Erreger der TSEs ist. Zweifel an der Prion-Theorie ergeben sich insbesondere aus der Beobachtung verschiedener TSEs-Erregerstämmen, die sich durch unterschiedliche Inkubationszeiten sowie durch unterschiedliche Zerstörungsmuster im Gehirn auszeichnen, da unklar ist, wie solche Stammeseigenschaften von einem einzigen Protein mit einer fest definierten Aminosäuresequenz codiert werden können.

Basierend auf der Prion-Theorie wird derzeit an der Entwicklung von Antikörpern für PrP^{Sc} gearbeitet, insbesondere an Antikörpern, die selektiv an PrP^{Sc}, nicht aber an PrP^{c} binden, um auf deren Grundlage neue Diagnoseverfahren für TSEs, beispielsweise basierend auf einem Immunoassay oder einem Immunoblot, zu etablieren. Bisher bekannte Verfahren beruhen auf Antikörpern, die eine Affinität für beide Spezies der Prion-Proteine, also sowohl für PrP^{c} als auch für PrP^{Sc} aufweisen, so dass in diesen Verfahren vor einer Antikörperreaktion das PrP^{c} proteolytisch degeneriert werden muss. Die Zuverlässigkeit der PrP^{Sc}-Detektion hängt somit in entscheidenden Maße von der Vorbehandlung ab. Ein solches Verfahren ist beispielsweise aus der WO-A-0 029 850 bekannt, bei dem zunächst die Prion-Proteine einer limitierten Proteolyse unterzogen werden, bevor PrP^{Sc} an einen ein N-terminales Epitop des PrP erkennenden, in einer Mikrotiterplatte immobilisierten Antikörper gebunden wird und mittels bekannter immunchemischer Verfahrensschritte quantifiziert wird. Ein Nachteil diesen Verfahren liegt darin, dass Prionenhaltiges Material der zu untersuchenden Individuen benötigt wird. Da beispielsweise bei BSE infizierten Rindern Prionen nur im zentralen Nervensystem vorhanden sind, solche Proben jedoch bei lebenden Rindern nicht bzw. nur unter erheblichen Aufwand, dann aber nur in sehr kleinen Mengen zu entnehmen sind, scheiden solche Verfahren zur Untersuchung lebender Rinder aus. Ob solche Verfahren jemals zur Diagnose der CJK bei Menschen geeignet sein werden, ist fraglich.

Aus der WO-A-9 813 694 ist ein alternatives Model zu der Prionen-Theorie über die Ursachen von TSEs bekannt, welches auf dem Phänomen der molekularen Mimikry aufbaut. Nach diesem Model sind BSE und die damit verwandten Krankheiten Autoimmunkrankheiten, die als Ergebnis der molekularen Homologie zwischen infektiösen Agenzien und dem Myelin der infizierten Individuen entstehen, so dass die gegen das infektiöse Agens gebildete Antikörper nicht nur mit diesem, sondern auch mit dem körpereigenen Myelin reagieren. Dieses Model basiert auf dem Ergebnis eines vor etwa 25 Jahren durchgeführten Experimentes, nach dem die Inokulation einer kurzen, thermoresistenten Sequenz eines Rindermyelinproteins bei Guinea Schweinen eine tödlich verlaufende und in ihren Symptomen BSE gleichenden Krankheit hervorrief. Auf Grundlage eines Sequenzvergleiches wird dort postuliert, dass einige Proteine von Mikroorganismen, beispielsweise aus *Acinetobacter calcoaceticus, Agrobacter tumefaciens* und *Ruminococcus albus* die nötige Homologie zu dem Rindermyelinprotein aufweist.

Nach diesem Model wird in der WO-A-9 813 694 ein Diagnoseverfahren für 'spongiforme Enzephalopathien' vorgeschlagen, bei dem Proben der zu überprüfenden Individuen auf solche Antikörper getestet werden, die an Antigen-Peptide binden, welche eine ausreichend hohe Homologie zu Myelin-Peptiden von Vertebraten aufweist. Dies kann in der Weise geschehen, dass die Blutseren der zu überprüfenden Individuen mit solchen Peptiden oder aber mit ganzen Bakterienzellen, welche solche Peptide ausreichender Homologie enthalten, wie beispielsweise *Acinetobacter calcoaceticus,* in Kontakt gebracht und die etwaig enthaltenden entsprechenden Antikörper beispielsweise mittels ELISA ausgewertet werden. Ähnliche Diagnoseverfahren werden in der WO-A-9 947 932, basierend auf IgA-Antikörpern, und der WO-A-0 031 545, basierend auf Antikörpern mit Affinität zu Myelin und/oder Myelin-Neurofilamenten, beschrieben. Da alle diese Diagnoseverfahren auf der nicht bewiesenen Annahme beruhen, dass die Antikörper gegen Myelin bzw. gegen die dem Myelin-Peptiden homologen Peptiden von *Acinetobacter calcoaceticus,* in unmitelbaren Zusammenhang mit der zu diagnostizierenden TSE stehen, ist bis zu einem Beweis der Richtigkeit der zugrunde liegenden Annahme diesen Verfahren keine verlässliche Aussagekraft zuzumessen.

Aufgabe der vorliegenden Erfindung ist es, ein Verfahren zur Erfassung der Pathogenese und zur Diagnose von 'transmissiblen spongiformen Enzephalopathien' bei Menschen und Vertebraten, insbesondere zur Diagnose der 'Bovinen Spongiformen Enzephalopathie' bei Rindern und zur Diagnose der 'Creutzfeldt-Jakob-Krankheit' bei Menschen, zur Verfügung zu stellen, welches zuverlässig ist und bei lebenden Menschen und Vertebraten angewendet werden kann.

Diese Aufgabe wird z.B. erfindungsgemäß durch ein Verfahren gelöst, bei welchem dem zu untersuchenden Individuum eine biologische Probe entnommen und an Neurotoxinen, insbesondere an neurotoxischen Mykotoxinen, und/oder deren Gehalt an neurotoxinbildenden Mikroorganismen, beispielsweise an Hefe oder an Schimmelpilzen, und/oder an Zink bestimmt wird.

Überraschenderweise wurde gefunden, dass die TSEs nicht durch das modifizierte Prion-Protein PrP^{Sc} hervorgerufen werden, sondern vielmehr in erster Linie durch einen Zinkmangel.

Zinkmangel führt bekanntermaßen zu einer Beeinträchtigung des Immunsystems. Des weiteren ist die wesentliche Bedeutung des als Cofaktor in mehr als 200 Enzymen vorkommenden Zinks sowohl für die Entwicklung als auch für die normale Funktionstätigkeit des Gehirns wissenschaftlich anerkannt. Als Cofaktor der Glutamat-Dehydrogenase (GDH), welche die Synthese des exzitatorischen Neurotransmitters Glutamat aus 2-Oxoglutarsäure und Ammoniumsalzen katalysiert, spielt Zink eine entscheidende Rolle bei der Regulation der exzitatorischen Neurotransmission. Durch Zinkmangel im Gehirn wird bekanntermaßen auch die Aktivität weiterer für den Stoffwechsel wichtiger Enzyme vermindert, beispielsweise die der Thymidinkinase, der Dopamin-β-Hydroxylase und der alkalische Phosphatase.

Die Bedeutung von Zink für den Stoffwechsel ist durch Tierexperimente belegt, bei denen durch eine künstlich herbeigeführte Senkung des Zinkgehaltes im Blutserum der Versuchstiere eine gesteigerte Reizbarkeit, Tremorzustände und eine Störung in der Bewegungskoordination in Form einer cerebellare Ataxie induziert wird (Henkin et al. 1975). Diese Befunde stehen in auffallender Ähnlichkeit zu den Symptomen von an BSE erkrankten Rindern. Eine weitere offensichtliche Analogie besteht zwischen der an BSE erkrankten Rindern zu beobachtenden Blendempfindlichkeit und der auf Zinkmangel zurückzuführenden Störung des Nachtsehens bei Menschen (A. Ochs, Fortschritte der Medizin 1998, S. 125 bis 129). Darüber hinaus ist bekannt, dass Zinkmangel beim Menschen mitunter auch tödlich verlaufende Krankheiten auslösen kann, wie beispielsweise Acrodermatitis enteropathica, eine autosomal rezessiv vererbbare Krankheit, die mit Hautveränderungen, Alopezie, Gedeih-und psychischen Störungen einhergeht und zu einer massiven Störung der zellulären Immunität führt. Eine komplette Remission dieser Krankheit, die in der Schulmedizin als klassisches Beispiel für Zinkmangel gilt und deren Symptome auffallende Ähnlichkeiten zu denen der Schafskrankheit "Scrapie" aufweisen, ist durch Zinksubstitution erreichbar (K. Grüngreiff, Med. Welt 1998, Seite 26).

Im Rahmen der Überlegungen, welche zu der vorliegenden Erfindung geführt haben, hat sich gezeigt, dass die 'transmissiblen spongiformen Enzephalopathien' durch Zinkmangel hervorgerufen werden. Wie Studien zeigen, ist Zink schon seit längerer Zeit nicht mehr in ausreichendem Maße in Böden vorhanden, so dass nicht nur das Vieh beim Weidegang sowie über die industriell hergestellten Futtermittel, sondern auch Menschen zu wenig Zink aufnehmen. Alternativ oder zusätzlich dazu kann Zinkmangel auch durch den Befall von Mikroorganismen, insbesondere durch den Befall von Schimmelpilzen, verursacht werden, da diese Mikroorganismen dem Wirtsorganismus das zum eigenen Wachstum benötigte Zink entziehen. Aufgrund der Auswirkungen auf das Immunsystem und der Auswirkungen auf das zentrale Nervensystem löst ein entsprechend hoher und über einen ausreichend langen Zeitrum aufrechterhaltener Zinkmangel die Symptome der TSE-Erkrankungen aus.

Nach den Erkenntnissen der vorliegenden Erfindung können die Auswirkungen von Zinkmangel durch Neurotoxine, insbesondere solchen von neurotoxinbildenden Mikroorganismen, in der Weise verstärkt werden, dass eine TSE bei gleicher Latenzzeit durch einen entsprechend geringeren Grad an Zinkmangel oder bei dem gleichen Grad an Zinkmangel innerhalb einer kürzeren Zeitspanne ausbricht. Neurotoxische Mykotoxine sind gegenüber hohen Temperaturen, den bekannten Desinfektionsmitteln, Proteasen und Strahlung überaus stabil, so dass sie auch gegenüber gängigen Sterilisationsverfahren resistent sind. Neurotoxinbildende Mikroorganismen, beispielsweise Schimmelpilze, sind aufgrund ihrer Anspruchslosigkeit gegenüber den Umgebungsbedingungen ubiquitär verbreitet und befallen insbesondere Getreide, Getreideprodukte und andere Futtermittel. Aufgrund des durch Zinkmangel geschwächten Immunsystems breiten sich die entsprechenden Mikroorganismen in den betreffenden Wirtorganismen schnell aus und geben daher eine erhöhte Menge an Neurotoxinen ab, die bekanntermaßen eine ganz besondere Affinität zu Nerven-, Binde- und Fettgewebe aufweisen. Des weiteren entziehen diese Mikroorganismen dem Wirtorganismus zusätzliches, für ihren eigenen Stoffwechsel benötigtes Zink. Über einen Zeitraum von mehreren Jahren führen die aufgrund des Zinkmangels im Gehirn hervorgerufenen Funktions- und Regulationsstörungen und die durch die Neurotoxine hervorgerufenen Gehirnschädigungen zu den für die TSEs-Erkrankungen typischen Symptomen.

Durch diese Erkenntnisse werden einerseits die langen Latenzzeiten bis zum Ausbruch der Krankheit und die hohe Stabilität der Erreger, andererseits aber auch die bisher unverstandene Existenz scheinbar verschiedener TSEs-Erregerstämmen, welche sich durch unterschiedliche Inkubationszeiten sowie durch unterschiedliche Zerstörungsmuster im Gehirn auszeichnen, erklärbar.

Gemäß einer ersten Ausführungsform der vorliegenden Erfindung erfolgt die Diagnose einer TSE-Infektion ausschließlich durch Bestimmung des Zinkgehaltes anhand einer oder mehrerer biologischer Proben. Die Bestimmung des Zinkgehaltes kann durch jedes dem Fachmann zu diesem Zweck bekannte quantitativ analytische Verfahren erfolgen. Als besonders genaues und zuverlässiges Verfahren hat sich in dem relevanten Konzentrationsbereich die Atomabsorptionsspektroskopie und die Neutronenaktivierungsanalyse erwiesen. Für besonders schnell durchzuführende Untersuchungen, für welche die dafür erforderlichen technischen Geräte nicht zur Verfügung stehen, kann die Konzentrationsbestimmung auch gravimetrisch oder photometrisch erfolgen.

Alternativ dazu kann der Zinkgehalt auch indirekt durch Messung der Konzentration an Enzymen, deren Aktivität bei einem vorliegenden Zinkmangel im Wesentlichen proportional zu dem Grad des Zinkmangels reduziert wird, bestimmt werden. Hierzu eignet sich vorzugsweise Carboxypeptidase, Carboanhydrase, Alkoholdehydrogenase sowie Thymidinkinase und besonders bevorzugt alkalische Phosphatase.

Als biologische Probe zur Zinkbestimmung eignen sich Haare, Urin, Fäkalien, Blutserum und besonders bevorzugt Vollblut, wobei für eine Zinkbestimmung anhand von Vollblut die Blutprobenentnahme aufgrund des im Vergleich zu dem Blutserum höheren erythrozytären Zinkgehaltes unter Vermeidung einer Hämolyse vorzunehmen ist. Für eine besonders zuverlässige Diagnose ist es vorteilhaft, den Zinkgehalt mehrer biologischer Proben verschiedenen Ursprungs aber aus ein und demselben Individuum zu bestimmen.

Eine sichere TSE-Diagnose liegt vor, wenn das relative Verhältnis des Zinkgehaltes der biologischen Probe des zu untersuchenden Individuums zu dem Mittelwert des Normalbereiches des Zinkgehaltes gleicher biologischer Proben gesunder Individuen derselben Art kleiner gleich 45% beträgt. Die Zinkkonzentration gesunder Vertebraten variiert zwischen den einzelnen Arten und ist insbesondere auch von der Art der biologischen Probe abhängig. Diese Werte können einerseits der einschlägiger Literatur entnommen werden oder anhand einer Mehrfachbestimmung an gesunden Individuen der zu untersuchenden Vertebraten-Art bestimmt werden.

Eine besonders zuverlässige Diagnose von TSE wird gemäß einer zweiten Ausführungsform der vorliegenden Erfindung ermöglicht, wenn für sich oder zusätzlich zu dem Zinkgehalt der Gehalt an Neurotoxinen, bevorzugt an solchen, die sich von neurotoxinbildenden Mikroorganismen ableiten, besonders bevorzugt an neurotoxischen Mykotoxinen und ganz besonders bevorzugt an einem oder mehreren Toxinen, die zu der Gruppe der Penitreme, Trichothecene, Ergot-Alkaloide und Aflatoxine gehören, bestimmt wird.

Für die Bestimmung der Neurotoxine können alle hierfür geeigneten Verfahren eingesetzt werden, wobei sich insbesondere die quantitative Massenspektroskopie als zuverlässiges Verfahren in Frage kommt. Sofern Antikörper gegen die zu untersuchenden Neurotoxine vorliegen, kann die Bestimmung auch über immunhistochemische Verfahren oder über Immunoassays erfolgen. Als biologische Proben sind vorzugsweise Gewebeproben des Binde- und Fettgewebes geeignet, da sich die Neurotoxine neben dem Nervengewebe insbesondere in diesen Gewebearten anreichern. Des weiteren kann die Neurotoxinmessung bevorzugt auch an geeigneten Mengen von Blut oder Urin vorgenommen werden.

Für die Diagnose einer TSE-Infektion ist weniger die Menge eines einzelnen Neurotoxins im Körper des untersuchten Individuums als vielmehr die Anzahl der insgesamt aufkommenden Neurotoxine entscheidend. Sofern das relative Verhältnis des Zinkgehaltes des zu untersuchenden Individuums kleiner gleich 45% beträgt, reicht für eine sichere Diagnose das Auffinden wenigstens eines Neurotoxins aus. Des weiteren liegt eine TSE-Infektion vor, wenn das relativen Verhältnis der Zinkkonzentration in dem Bereich zwischen 46 und 60 % liegt und zusätzlich wenigstens zwei verschiedene Neurotoxine nachweisbar sind.

Anstelle oder zusätzlich zu der Bestimmung von Neurotoxinen kann gemäß einer dritten Ausführungsform der vorliegenden Erfindung im Rahmen des erfindungsgemäßen Diagnoseverfahrens auch eine Bestimmung des Gehaltes an neurotoxinbildenden Mikroorganismen erfolgen. Bei einer solchen Bestimmung ist vornehmlich auf diejenigen Mikroorganismen abzustellen, die aufgrund ihres Vorkommens am ehesten mit dem zu untersuchenden Vertebraten in Kontakt kommen, vorzugsweise auf Hefen und/oder Schimmelpilze. Aufgrund ihres Vorkommens ist insbesondere bei der Untersuchung von Rindern auf eine mögliche BSE-Infektion oder von Menschen auf eine etwaige CJK-Infektion besonders bevorzugt der Gehalt an Schimmelpilzen der Gattung *Aspergillus,* insbesondere an *Aspergillus fumigatus, Aspergillus niger, Aspergillus flavus* und/oder *Aspergillus stellatus,* an Hefen der Gattung *Candida,* insbesondere an *Candida albicans, Candida glabrata, Candida tropicalis,* und/oder *Candida parapsilosis* und/oder aufgrund der Toxizität der von diesen gebildeten Neurotoxine an *Claviceps purpurea* und/oder an *Emericella variecolor* zu bestimmen.

Die Charakterisierung der neurotoxinbildenden Mikroorganismen kann mittels der bekannten mikrobiologischen Standardverfahren erfolgen. Beispielsweise kann die Bestimmung der Mikroorganismen in der Weise erfolgen, dass die biologische Probe in verschiedenen Verdünnungen auf Agarplatten eines geeigneten Nährmediums ausgestrichen werden und die nach entsprechender Inkubation erhaltenen Zellklone ggf. nach Anfärben optisch oder mittels molekularbiologischer Standardverfahren, z.B. unter Verwendung für die zu bestimmenden Mikroorganismen spezifischen Sonden durch Southern-, Northern- oder Western-blot Analyse oder auch durch Koloniehybridisierung, charakterisiert werden. Die Bestimmung der Mikroorganismen kann selbstverständlich auch durch jede andere bekannte, beispielsweise immunchemische Methoden unter Verwendung für die zu bestimmenden Mikroorganismen spezifischer Antikörper, insbesondere Antikörper gegen spezifischen Zellmembranrezeptoren, erfolgen.

Zur Bestimmung der neurotoxinbildenden Mikroorganismen eignen sich als biologische Proben insbesondere Fäkalien, auch Tupferproben aus Maul und After, Gewebe, Blut und Urin. Um Negativproben schnell und ohne großen experimentellen Aufwand ermitteln zu können, erfolgt die Bestimmung vorzugsweise in der Weise, dass zunächst Fäkalien des zu untersuchenden Individuums auf die Anwesenheit von Mikroorganismen jeglicher Art geprüft werden. Nur bei einem positiven Befund erfolgt anschließend die eigentliche Untersuchung von Blut oder Urin bezüglich der vorstehend aufgeführten Mikroorganismen.

Eine sichere TSE-Diagnose liegt vor, wenn das relative Zinkverhältnis kleiner gleich 45% beträgt und wenigstens ein Mikroorganismus aus der Gruppe, die aus *Aspergillus fumigatus, Aspergillus niger, Aspergillus flavus, Aspergillus stellatus, Candida albicans, Candida glabrata, Candida tropicalis, Candida parapsilosis, Claviceps purpurea* und *Emericella variecolor* besteht, oder ein anderer neurotoxinbildender Mikroorganismus gefunden wurde. Bei einem relativen Zinkverhältnis zwischen 46 und 60% kann eine TSE-Infektion sicher diagnostiziert werden, sofern wenigstens zwei der vorstehend genannten Mikroorganismen in der biologischen Probe nachweisbar sind.

Besonders zuverlässige Ergebnisse werden mit einem der Verfahren gemäß der vorliegenden Erfindung erhalten, wenn die Bestimmung der Neurotoxinkonzentration und/oder die Bestimmung des Gehalts an neurotoxinbildenden Mikroorganismen und/oder die Bestimmung des relativen Zinkverhältnisses an wenigstens zwei biologischen Proben unterschiedlicher Natur erfolgen.

## Patentansprüche

1. Verfahren zur Erfassung der Pathogenese und/oder zur Diagnose von bei Menschen und Vertebraten auftretenden 'transmissiblen spongiformen Enzephalopathien', insbesondere zur Diagnose der bei Rindern auftretenden 'Bovinen Spongiformen Enzephalopathie' und zur Diagnose der bei Menschen auftretenden 'Creutzfeldt-Jakob-Krankheit', **dadurch gekennzeichnet, dass** der Gehalt wenigstens einer dem zu untersuchenden Individuum entnommenen biologischen Probe an Neurotoxinen und/oder an neurotoxinbildenden Mikroorganismen und/oder an Zink bestimmt wird.

2. Verfahren nach Anspruch 1, **dadurch gekennzeichnet, dass** der Gehalt an Zink und an Neurotoxinen und/oder an neurotoxinbildenden Mikroorganismen bestimmt wird.

3. Verfahren nach Anspruch 1 oder 2, **dadurch gekennzeichnet, dass** der Gehalt an Zink mittelbar durch Messen der Konzentration an Carboxypeptidase, Carboanhydrase, Alkoholdehydrogenase, Thymidinkinase und/oder besonders bevorzugt an alkalische Phosphatase bestimmt wird.

4. Verfahren nach einem der Ansprüche 1 bis 3, **dadurch gekennzeichnet, dass** als biologische Probe Haare, Urin, Fäkalien, Tupferproben, Gewebe, Blutserum und besonders bevorzugt Vollblut verwendet werden.

5. Verfahren nach einem der Ansprüche 1, 2 oder 4, **dadurch gekennzeichnet, dass** der Gehalt an Zink durch Gravimetrie, Spektroskopie oder Photometrie und bevorzugt durch Atomabsorptionsspektroskopie oder Neutronenaktivierungsanalyse bestimmt wird.

6. Verfahren nach einem der Ansprüche 1 bis 5, **dadurch gekennzeichnet, dass** der Gehalt an Neurotoxinen, die von neurotoxinbildenden Mikroorganismen gebildet werden, und bevorzugt der Gehalt an neurotoxischen Mykotoxinen bestimmt wird.

7. Verfahren nach Anspruch 6, **dadurch gekennzeichnet, dass** der Gehalt an Penitremen, Trichothecenen, Ergot-Alkaloiden und/oder Aflatoxinen bestimmt wird.

8. Verfahren nach einem der Ansprüche 1 bis 7, **dadurch gekennzeichnet, dass** der Gehalt an Neurotoxinen durch chemische, physikalische oder biochemische Methoden bestimmt wird.

9. Verfahren nach Anspruch 8, **dadurch gekennzeichnet, dass** der Gehalt an Neurotoxinen durch spektroskopische Verfahren, bevorzugt Massenspektroskopie, durch histochemische Verfahren, bevorzugt immunhistochemische Verfahren, oder durch immunchemische Verfahren, bevorzugt Immunoassays, erfolgt.

10. Verfahren nach einem der Ansprüche 1 bis 9, **dadurch gekennzeichnet, dass** der Gehalt an Hefen oder Schimmelpilzen, bevorzugt der Gehalt an Schimmelpilzen der Gattung *Aspergillus* und/oder an Hefen der Gattung *Candi*da und besonders bevorzugt der Gehalt an *Aspergillus fumigatus, Aspergillus niger, Aspergillus flavus, Aspergillus stellatus, Candida albicans, Candida glabrata, Candida tropicalis, Candida parapsilosis* und/oder *Claviceps purpurea* und/oder *Emericella variecolor* bestimmt wird.

11. Verfahren nach einem der Ansprüche 1 bis 10, **dadurch gekennzeichnet, dass** die Charakterisierung der Mikroorganismen durch mikrobiologische oder molekularbiologische Standardverfahren, beispielsweise Koloniehybridisierung, erfolgt.

12. Verfahren nach einem der Ansprüche 1 bis 11, **dadurch gekennzeichnet, dass** wenigstens zwei verschiedene Arten von biologischen Proben entnommen und untersucht werden.

## Claims

1. A method of determining the pathogenesis and/or of diagnosing "transmissible spongiform encephalopathies" in man and vertebrates, especially for diagnosing "bovine spongiform encephalopathy" in cattle and "Creutzfeldt-Jakob disease" in man, **characterized in that** the content of neurotoxins and/or of neurotoxin-forming microorganisms and/or of zinc in at least one biological sample taken from the individual to be investigated is determined.

2. A method according to Claim 1, **characterized in that** the content of zinc and of neurotoxins and/or of neurotoxin-forming microorganisms is determined.

3. A method according to Claim 1 or 2, **characterized in that** the content of zinc is determined indirectly by measuring the concentration of carboxypeptidase, carboanhydrase, alcohol dehydrogenase, thymidine kinase and/or, particularly preferred, alkaline phosphatase.

4. A method according to one of Claims 1 to 3, **characterized in that** hair, urine, faecal matter, swabs, tissue, blood serum and, particularly preferred, whole blood are used as biological samples.

5. A method according to one of Claims 1, 2 or 4, **characterized in that** the zinc content is determined by gravimetry, spectroscopy of photometry and preferably by atomic absorption spectroscopy or neutron activation analysis.

6. A method according to one of Claims 1 to 5, **characterized in that** the content of neurotoxins, which are formed by neurotoxin-forming microorganisms, and preferably the content of neurotoxic mycotoxins is determined.

7. A method according to Claim 6, **characterized in that** the content of penitrems, trichothecenes, ergot alkaloids and/or aflatoxins is determined.

8. A method according to one of Claims 1 to 7, **characterized in that** the content of neurotoxins is determined by chemical, physical or biochemical methods.

9. A method according to Claim 8, **characterized in that** the content of neurotoxins is determined by spectroscopic methods, preferably mass spectroscopy, by histochemical methods, preferably immuno-histochemical methods, or by immunochemical methods, preferably immunoassays.

10. A method according to one of Claims 1 to 9, **characterized in that** the content of yeasts or mould fungi is determined, preferably the content of mould fungi of the species *Aspergillus* and/or of yeasts of the species *Candida* and, particularly preferred, the content of *Aspergillus fumigatus, Aspergillus niger, Aspergillus flavus, Aspergillus stellatus, Candida albicans, Candida glabrata, Candida tropicalis, Candida parapsilosis* and/or *Claviceps purpurea* and/or *Emericella variecolor.*

11. A method according to one of Claims 1 to 10, **characterized in that** the microorganisms are **characterized by** using standard microbiological or molecular biological methods, for example colony hybridization.

12. A method according to one of Claims 1 to 11, **characterized in that** at least two different types of biological sample are taken and investigated.

## Revendications

1. Procédé pour la détection de la pathogenèse et/ou pour le diagnostic des « encéphalopathies spongiformes transmissibles » se développant chez l'homme et les vertébrés, notamment pour le diagnostic de l' « encéphalopathie spongiforme bovine » se développant chez les bovins et pour le diagnostic de la « maladie de Creutzfeldt Jakob » se développant chez l'homme, **caractérisé en ce qu'**on détermine la teneur en neurotoxines et/ou en microorganismes formant des neurotoxines et/ou en zinc d'au moins un des échantillons biologiques prélevés chez l'individu à examiner.

2. Procédé selon la revendication 1, **caractérisé en ce qu'**on détermine la teneur en zinc et en neurotoxines et/ou en microorganismes formant des neurotoxines.

3. Procédé selon la revendication 1 ou 2, **caractérisé en ce que** la teneur en zinc est déterminée indirectement en mesurant la concentration en carboxypeptidase, en carboanhydrase, en alcooldéhydrogénase, en thyminidase et/ou de façon particulièrement privilégiée en phosphatase alcaline.

4. Procédé selon l'une des revendications 1 à 3, **caractérisé en ce qu'**on utilise comme échantillons biologiques des cheveux, de l'urine, des matières fécales, des échantillons par écouvillon, des tissus, du sérum sanguin et de façon particulièrement privilégiée de sang entier.

5. Procédé selon l'une des revendications 1, 2 ou 4, **caractérisé en ce que** la teneur en zinc est déterminée par gravimétrie, par spectroscopie ou par photométrie et de façon particulièrement privilégiée par spectroscopie par absorption atomique ou par analyse par activation des neutrons.

6. Procédé selon l'une des revendications 1 à 5, **caractérisé en ce qu'**on détermine le teneur en neurotoxines qui sont formées par des microorganismes formant des neurotoxines, et de façon privilégiée la teneur en mycotoxines neurotoxiques.

7. Procédé selon la revendication 6, **caractérisé en ce qu'**on détermine la teneur en pénitrem, en trichothécène, en alcaloïde de l'ergot et/ou en aflatoxines.

8. Procédé selon l'une des revendications 1 à 7, **caractérisé en ce que** la teneur en neurotoxines est déterminée par des méthodes chimiques, physiques ou biochimiques.

9. Procédé selon la revendication 8, **caractérisé en ce que** la teneur en neurotoxines est déterminée par des procédés spectroscopiques, de préférence par spectroscopie de masse, par des procédés histochimiques, de préférence par des procédés immunohistochimiques, ou par des procédés immunochimiques, de préférence par immunoessais.

10. Procédé selon l'une des revendications 1 à 9, **caractérisé en ce qu'**on détermine la teneur en levures ou en champignons de moisissure, de préférence la teneur en champignons de moisissure de l'espèce *Aspergillus* et/ou en levure de l'espèce *Candida* et de préférence la teneur en *Aspergillus fumigatus, Aspergillus niger, Aspergillus flavus, Aspergillus stellatus, Candida albicans, Candida glabrata, Candida tropicalis, Candida parapsilosis* et/ou *Claviceps purpurea* et/ou *Emericella variecolor.*

11. Procédé selon l'une des revendications 1 à 10, **caractérisé en ce que** la caractérisation des microorganismes est réalisée par des procédés standards de microbiologie ou de biologie moléculaire, par exemple par hybridation de colonies.

12. Procédé selon l'une des revendications 1 à 11, **caractérisé en ce qu'**au moins deux types d'échantillons biologiques différents sont prélevés et analysés.
